# EUROPÄISCHE PATENTSCHRIFT

(11) **EP 4 588 461 B1**
(45) Veröffentlichungstag und Bekanntmachung des Hinweises auf die Patenterteilung: **25.11.2026**
(21) Anmeldenummer: 25181363.0
(22) Anmeldetag: 15.09.2022
(51) Int. Cl.: A61F 9/008

(54) **KORREKTUR DER REFRAKTION EINES AUGES DURCH HORNHAUTMODIFIKATION**
CORRECTING THE REFRACTION OF AN EYE BY CORNEAL MODIFICATION
CORRECTION DE LA RÉFRACTION D'UN OEIL PAR MODIFICATION DE LA CORNÉE

(30) Priorität: 17.09.2021 DE 102021124087
(43) Veröffentlichungstag der Anmeldung: 23.07.2025
(62) Dokumentnummer(n) der früheren Anmeldung(en) nach Art. 76 EPÜ: 22789882.2 / 4 401 689
(73) Patentinhaber: Carl Zeiss Meditec AG, 07745 Jena (DE)
(72) Erfinder: BISCHOFF, Mark, 07745 Jena (DE); KINDT, Johannes, 99425 Weimar (DE)
(74) Vertreter: Patentanwälte Geyer, Fehners & Partner mbB

(56) Entgegenhaltungen:
- WO-A1-2012/035403
- DE-A1- 102013 218 415
- US-A1- 2017 027 754

## Beschreibung

Die Erfindung bezieht sich auf ein Verfahren zur Korrektur der Refraktion eines Auges mittels Hornhautmodifikation sowie eine entsprechende Vorrichtung.

Die menschliche Fehlsichtigkeit wird seit alters her durch Vorsatzlinsen in Form von Brillen korrigiert. In jüngster Zeit gibt es diverse Ansätze, die Fehlsichtigkeit des Auges dadurch zu korrigieren, dass die Hornhaut modifiziert wird. Die Modifikation soll dafür sorgen, dass sich die Krümmung der Hornhaut ändert. Zur Korrektur der Myopie muss die Hornhautvorderfläche abgeflacht werden, weshalb das zu entnehmende Volumen in der Mitte, d.h. im Bereich der Sehachse dicker ist, als am Rand. Zur Korrektur der Hyperopie muss die Hornhaut hingegen an der Vorderfläche stärker gekrümmt werden, weshalb das zu entnehmende Volumen am Rand dicker ist, als in der Mitte. Die Gesamtabbildungseigenschaften des Auges werden dadurch so beeinflusst, dass eine Fehlsichtigkeit gemindert oder im Idealfall sogar gänzlich ausgeglichen wird.

Ein diesbezüglich sehr erfolgreiches Verfahren wurde von der Carl Zeiss Meditec AG unter der Bezeichnung SMILE entwickelt. Es isoliert mittels gepulster Laserstrahlung ein Lentikel in der Hornhaut, das anschließend durch einen seitlichen Öffnungsschnitt, der zur Augenhornhautoberfläche führt und als Arbeitskanal dient, aus der Hornhaut entfernt werden kann. Das Volumen des Lentikels ist so strukturiert und bemessen, dass die Vorderfläche der Hornhaut ihre Krümmung wie zur Korrektur nötig ändert. Das Verfahren bewirkt eine subtraktive Korrektur, da Volumen entfernt wird.

Ein anderer Ansatz fügt in die Augenhornhaut Implantate ein. Dazu wird in die Hornhaut ein Schlitz eingebracht, in den das Implantat eingeschoben wird. Es ist so gestaltet, dass es die Krümmung der Hornhautvorderfläche auf gewünschte Weise ändert. Dieser Ansatz bewirkt somit eine additive Korrektur, da Volumen hinzugefügt wird. Die WO 2016/050711 A1 behebt ein Problem dieser additiven Korrektur, das darin liegt, dass am Rand des Implantats in der Augenhornhaut Spannungen auftreten. Um diese zu lösen, wird das Eingliedern des Implantats in die Augenhornhaut zusätzlich durch Entlastungsschnitte in der Augenhornhaut unterstützt.

Gemäß US 5722971 A wird eine als "Pocket" bezeichnete Kammer geschaffen, in welche ein festes oder teilfestes Material eingefügt werden kann. Hierfür werden drei Varianten erwähnt. In einer ersten Variante überfüllt das feste oder teilfeste Material die "Pocket; es liegt eine additive Korrektur vor. In einer zweiten Variante, unterfüllt das feste oder teilfeste Material die "Pocket; es liegt im Ergebnis eine subtraktive Korrektur vor, da nach dem Eingriff in der Hornhaut weniger Material vorhanden ist, als zuvor. In einer dritten Variante füllt das feste oder teilfeste Material diese "Pocket" genau aus. Die Veränderung ist volumenneutral und eine Modifikation der optischen Wirkung der Hornhaut kann damit nur erreicht werden, wenn das feste oder teilfeste Material einen anderen Brechungsindex als die Hornhaut aufweist. Die Schrift sieht es vor, nach dem Schaffen der Kammer das Auge zu vermessen, um dann festzulegen, welches Implantat eingefügt werden soll.

Ebenfalls das Einbringen von Material in die Augenhornhaut findet sich in der WO 2008/131888 A1, dort zum Zwecke der Transplantation, d.h. zum Austausch beschädigter, beispielsweise trüber Augenhornhaut. Mittels Laserstrahlung wird der zu ersetzende Teil der Augenhornhaut umgrenzt und durch einen ebenfalls mit Laserstrahlung geschaffenen Öffnungsschnitt entnommen. In die derart entstandene Kammer wird ein Spendermaterial eingefügt, so dass der schadhafte Teil der Augenhornhaut ausgetauscht ist. Damit ist per so noch keine additive Korrektur erreicht, wenn das Spendermaterial die bestehende Hornhaut 1:1 ersetzt. Es ist in der genannten Publikation aber auch erwähnt, dass das einzufügende Spendermaterial in seinen Abmessungen etwas größer oder kleiner sein kann als die Kammer, um zugleich eine bestehende Fehlsichtigkeit zu korrigieren, also die Krümmung der Hornhautvorderseite günstig zu beeinflussen. Ist das einzufügende Spendermaterial in seinen Abmessungen etwas größer, wird eine additive Korrektur bewirkt, ist es kleiner, eine subtraktive.

Die DE 102013218415 A1 befasst sich mit der Eingliederung eines Presbyopie-Implantats, das zur Korrektur der Altersweitsichtigkeit (Presbyopie) vorgesehen ist. Es hat den Zweck, den Durchtritt der Strahlung durch die Augenhornhaut in sehachsennahen Bereichen anders zu gestalten, als in sehachsenfernen Bereichen, und soll die Tiefenschärfe erhöhen. Die DE 102013218415 A1 sieht insbesondere zylinderförmige Vorsprünge vor, die ausgebildet werden, um das Presbyopie-Implantat in der Augenhornhaut gegen Verrutschen zu sichern. Dieses Presbyopie-Implantat ist in einem Beispiel als ringartige Scheibe ausgebildet, welche die Tiefenschärfe der optischen Abbildung verbessert, was die Altersweitsichtigkeit günstig beeinflusst. Insofern wird dieser Druckschrift ein Lentikelextraktionsverfahren, wie bei SMILE, mit einem Implantationsverfahren kombiniert, bei dem im Anschluss an die Lentikelentnahme ein Implantat in die Hornhaut platziert wird. Allerdings wird eine Refraktionskorrekturwirkung durch das Implantat in DE 102013218415 A1 nicht weiter thematisiert; es wird vielmehr diskutiert, dass eine über die Presbyopie hinausgehende Korrekturwirkung durch die Extraktion eines Lentikels entsteht. Zur Refraktionskorrektur sieht diese Schrift also einen subtraktiven Ansatz vor.

Der Erfindung liegt die Aufgabe zugrunde, ein Verfahren zur Ermittlung von Steuerdaten zur Korrektur der Refraktion eines Auges durch Hornhautmodifikation sowie eine entsprechende Vorrichtung anzugeben, mit der die Nachteile des Standes der Technik behoben sind, insbesondere eine möglichst zuverlässige Fehlsichtigkeitskorrektur über einen weiten Dioptrienbereich, insbesondere im Falle der Hyperopie, zu gewährleisten.

Die Erfindung ist in den unabhängigen Ansprüchen gekennzeichnet. Die abhängigen Ansprüche betreffen bevorzugte Weiterbildungen.

Die Erfindung kombiniert die Einfügung eines Implantats in die Augenhornhaut - ein additives Korrekturverfahren - mit der Entnahme eines Lentikels - einem subtraktiven Korrekturverfahren. Dabei wird ausgehend von Daten über einen Refraktionskorrekturbedarf des Auges zuerst ein Implantat hinsichtlich des von ihm bewirkten additiven Refraktionsveränderungswertes bestimmt, das in die Hornhaut eingesetzt werden soll. Dieser additive Refraktionsveränderungswert ist, z.B. durch die Abmessungen und die räumliche Gestaltung des Implantats, klar definiert und einfach zu berechnen oder für ein Implantat bekannt. Er korrigiert jedoch in keiner Ausführungsform die Fehlsichtigkeit vollständig. Die nachfolgend erläuterten drei Varianten beziehen sich auf Aspekte, welche mit dieser nur unvollständigen Korrektur, die durch den additiven Refraktionsveränderungswert erreicht wird, zusammenhängen. Liegt das Implantat zumindest hinsichtlich dessen additiven Refraktionsveränderungswerts fest, wird ein subtraktiver Refraktionsveränderungswert berechnet. Dieser entspricht dem Unterschied zwischen dem insgesamt nötigen Refraktionskorrekturbedarf und dem zuvor bestimmten additiven Refraktionsveränderungswert. Anschließend wird auf Basis des subtraktiven Refraktionsveränderungswertes ein in der Hornhaut zu isolierendes Lentikel derart berechnet, dass es durch Entfernen oder nach der Entnahme (für sich) aus der Hornhaut den subtraktiven Refraktionsveränderungswert bewirkt. Abschließend wird eine Schnittfläche berechnet, die das Lentikel in der Hornhaut isoliert und damit eine spätere Entnahme vorbereitet. Dieser isolierende Schnitt umgrenzt gleichzeitig eine Kammer für das einzusetzende Implantat, die dann leer ist, wenn das Lentikel entnommen wurde.

Die Erfindung geht also bei einer Kombination von additiver Fehlsichtigkeitskorrektur (Einsetzen eines refraktionsverändernden Implantats) und subtraktiver Fehlsichtigkeitskorrektur (Entnahme eines refraktionsverändernden Lentikels) erstmals von der Refraktionsveränderung aus, welche das Implantat verursacht, wenn es in die Hornhaut eingesetzt ist. Auf Basis dieser Refraktionsveränderung wird eine subtraktive Refraktionskorrektur berechnet, welche vom Lentikel zu leisten ist. Im Stand der Technik ist dieser Ansatz nicht verfolgt worden, da dort zur Refraktionsveränderung entweder ausschließlich mit additiver oder subtraktiver Fehlsichtigkeitskorrektur gearbeitet wurde (Implantat gemäß WO 2016/050711 A1; Lentikelentnahme gemäß DE 102013218415 A1), die Wechselwirkung zwischen additiver und subtraktiver Fehlsichtigkeitskorrektur nicht weiter analysiert wurde (US 5722971 A) oder mit der Entnahme des Lentikels überhaupt keine Refraktionsänderung beabsichtigt oder verbunden ist (Transplantationsansatz der WO 2008/131888 A1).

Die Kombination aus additiver und subtraktiver Refraktionsbeeinflussung und das Ausgehen vom additiven Refraktionsveränderungswert bzw. dem Implantat haben besondere und überraschende Vorteile. Es ist nicht mehr problematisch, dass eine ausschließlich subtraktive Korrektur nach dem Entfernen bzw. der Entnahme eines Lentikels, das bei starken Korrekturen am Rand oder im Zentrum sehr dick sein kann, zur Unterschreitung einer für eine ausreichende Stabilität kritischen minimalen Dicke führen kann. Diese Einschränkung, welche mit subtraktiven Verfahren verbunden ist, kommt sowohl bei Korrekturen der Weitsichtigkeit als auch der Kurzsichtigkeit zum Tragen, ist jedoch bei der Weitsichtigkeitskorrektur besonders problematisch, da die maximale Dicke des zu entnehmenden Lentikels dort am Rand des Lentikels liegt, was physiologisch ungünstig ist. Dort entsteht die Problematik einer Art Stufe, wenn das Lentikel entnommen wurde. Die Kombination von additiver und subtraktiver Fehlsichtigkeitskorrektur kann dies vermeiden.

Ein weiterer Vorteil ergibt sich in einer ersten Variante, wenn der additive Refraktionsveränderungswert bzw. das Implantat derart bestimmt wird, dass er/es eine Überkorrektur der Hyperopie oder Myopie bewirkt. Unter Überkorrektur wird dabei verstanden, dass eine bestehende Hyperopie oder Myopie nicht nur ausgeglichen, sondern (wenn nur das Implantat verwendet würde) in das Gegenteil, nämlich in eine Myopie oder Hyperopie verwandelt würde. Die Korrektur ist insofern zu stark. Dies tritt jedoch im Ergebnis nicht ein, da die subtraktive Restrefraktionskorrektur eine entsprechende gegenläufige Myopie- bzw. Hyperopiekorrektur umfasst. Auf den ersten Blick erscheint es widersinnig, mit irgendeiner Korrektur, sei es additiv oder subtraktiv, eine Überkorrektur auszuführen, also mehr Material einzufügen oder zu entnehmen, als eigentlich für die Korrektur nötig wäre. Die Überkorrektur durch den additiven Refraktionsveränderungswert bzw. das Implantat hat jedoch den großen Vorteil, dass die Grundgeometrie des Implantats der des zu entnehmenden Lentikels gleicht. Im Falle der Hyperopie hat das einzufügende Implantat seine maximale Dicke an der Mitte und ist am Rand, also in achsenfernen Bereichen dünner, da die Hornhautvorderfläche stärker gekrümmt werden soll. Aufgrund der Überkorrektur würde das Implantat für sich alleine eine zu große Krümmungssteigerung bewirken. Dies wird durch den subtraktiven Refraktionsveränderungswert bzw. das Lentikel kompensiert, das deshalb ebenfalls ein Volumen hat, welches in der Mitte dicker ist, als am Rand. In einer Schnittansicht haben damit das Implantat und das Lentikel dieselbe Grundform mit größerer Dicke im Zentrum und geringerer Dicke am Rand. Die durch die Lentikelentnahme bereitgestellte Kammer hat dann eine Grundform, die für die Aufnahme des Implantats ideal ist. Analoges gilt im inversen Falle einer Myopiekorrektur, bei der sowohl das Implantat, als auch, aufgrund der nötigen Überkorrekturkompensierung, das Lentikel im Zentrum dünner ist, als am Rand. Das auf den ersten Blick widersinnig erscheinende Vorgehen erleichtert damit die Einfügung des Implantats, da die durch das Lentikel bereitgestellte Kammer die gleiche Grundform wie das Implantat hat. Natürlich sind die einzelnen Abmessungen zwischen Lentikel und Implantat nicht identisch, jedoch erleichtert die Übereinstimmung in der Grundform das Einfügen erheblich und vermeidet insbesondere Spannungen am Rand der Kammer, wie sie Gegenstand der WO 2016/050711 A1 sind.

Dies gilt ganz besonders, wenn das Volumen des Implantats nicht größer ist, als das des Lentikels. Das kann durch geeignete Wahl der beiden Refraktionsveränderungswerte einfach eingestellt werden.

Eine zweite Variante hat den Vorteil, dass nunmehr eine Rotationsstellung des Implantats auch bei einer rotationsasymmetrischen, insbesondere astigmatischen Korrektur keine Probleme mehr bereitet. Herkömmliche Ansätze müssen hier beim Einfügen des dann nicht rotationssymmetrischen Implantates sicherstellen, dass dessen Rotationslage zur Orientierung (Achslage) des Astigmatismus passt. Weiter musste Vorsorge dafür getroffen werden, dass das Implantat im Laufe der Zeit nicht unerwünscht aus dieser vorbestimmten Lage herausrotiert. Die Wahl der beiden Refraktionsveränderungswerte, also der Refraktionsveränderung durch das Implantat und der Refraktionsveränderung durch das Lentikel, erlaubt es nun, ein rein rotationssymmetrisches Implantat vorzusehen, das unabhängig von seiner Drehstellung in der Augenhornhaut dieselbe Refraktionsveränderung zur Folge hat. Die insgesamt nötige Rotationsasymmetrie wird ausschließlich durch das daraufhin ermittelte Lentikel, d.h. den subtraktiven Refraktionsveränderungswert bewirkt. Da das Lentikel durch eine Schnittfläche in der Augenhornhaut isoliert und letztlich definiert wird, ist diese Rotationsasymmetrie sehr einfach einzustellen. Astigmatische Korrekturen sind beispielsweise für das SMILE-Verfahren gut bekannt. Die Kammer, in welche das rotationssymmetrische Implantat eingesetzt wird, ist rotationsasymmetrisch, was sich aber über die Zeit hinweg nicht ändern kann, selbst wenn das Implantat sich in der Kammer verdrehen sollte. Der erfindungsgemäße Startpunkt von der Refraktionsveränderung, die durch das Implantat erreicht wird, erlaubt somit sehr einfach auch rotationsasymmetrische Korrekturen zu beherrschen, ohne dass besondere Vorkehrungen für die Verankerung des Implantats gegen rotierende Lageveränderungen in der Kammer getroffen werden müssten.

Das einzusetzende Implantat ist bevorzugt multifokal, um z.B. eine Presbyopie-Korrektur zu bewirken. Unter Presbyopie versteht man die altersbedingte Weitsichtigkeit, welche durch den Verlust der Akkommodation hervorgerufen wird. Ein Presbyopie-korrigierendes multifokales Implantat ist beispielsweise aus WO 2021/156203 A1 bekannt. Dieses umfasst einen Linsenkörper mit zwei konzentrischen Zonen, die unterschiedliche Beugungsstrukturen zueinander aufweisen. Die Beugungsstrukturen sind so ausgebildet, dass sie mehrere Brennpunkte für bestimmte Wellenlängen im Bereich des sichtbaren Lichts bereitstellen.

Die Verwendung von Implantaten ging bislang immer davon aus, dass die Implantate im Wesentlichen für Patienten individuell angefertigt werden müssen, um eine vollständige Korrektur bzw. eine angestrebte Hornhautmodifikation vollständig zu erreichen. Der Startpunkt bei der Refraktionsveränderung des Implantats und das Anpassen des Lentikels auf die verbleibende Restrefraktionskorrektur erlaubt es nun in einer weiteren Variante, mit standardisierten Implantaten zu arbeiten. Es ist hierfür vorgesehen, ein Set aus Implantaten bereitzustellen, die jeweils zur Refraktionsveränderung durch Einsetzen in die Hornhaut ausgebildet sind, wobei das Set mehrere Implantate mit jeweils individuellem additivem Refraktionsveränderungswert aufweist. Das Bestimmen des additiven Refraktionsveränderungswertes umfasst dann das Auswählen eines dieser Implantate aus dem Set. Dieser Ansatz erlaubt eine industrielle Produktion von Implantaten. Dabei muss nicht mehr darauf geachtet werden, dass das verwendete Material für die Implantate eine nachträgliche patientenindividuelle Bearbeitung (im Labor oder in situ) möglich macht. Es können Materialien zum Einsatz kommen, die sehr viel besser auf die Biokompatibilität abgestimmt sind. Zweckmäßigerweise wird man beim Auswählen aus dem Set dasjenige der Implantate auswählen, dessen Refraktionsveränderung dem Refraktionskorrekturbedarf am nächsten liegt. Natürlich kann auch hier die Eigenschaft verwendet werden, dass das Implantat dabei eine Überkorrektur bewirkt und/oder rein rotationssymmetrisch ausgebildet ist, beispielsweise durch einen Satz aus sphärischen Implantaten.

Das geschilderte Verfahren zur Erzeugung von Steuerdaten umfasst die Vorbereitung zur Korrektur der Refraktion eines Auges durch Hornhautmodifikation und erfordert keinen chirurgischen Schritt. Es kann jedoch um einen solchen zu einem chirurgischen Verfahren ergänzt werden. In diesem Schritt wird eine Laservorrichtung zum Erzeugen der Schnittfläche verwendet, das Lentikel wird aus der Hornhaut entnommen, und das Implantat wird in die nach Entnahme des Lentikels verbleibende Kammer in der Hornhaut eingefügt. Das Verfahren kann dabei und ganz grundsätzlich mit einem Computer, insbesondere umfassend einen Prozessor, ausgeführt werden. Dieser Computer kann als Planungsstation ausgebildet werden, wie es anderweitig im Stand der Technik bekannt ist.

Die Erfindung erfasst diesbezüglich weiter ein Softwareprodukt zum Durchführen des genannten Verfahrens, da die Berücksichtigung des Refraktionskorrekturbedarfs, das Bestimmen des Implantats sowie das Berechnen der Restrefraktionskorrektur und der Schnittfläche von einer entsprechenden Software unproblematisch ausgeführt werden kann.

Die zur Lösung der Aufgabe vorgesehene Vorrichtung entspricht im Ansatz dem geschilderten Verfahren, wobei eine Berechnungseinrichtung vorgesehen ist, welche zur Ausführung der entsprechenden Verfahrensschritte konfiguriert ist.

Soweit vorstehend von sphärischen bzw. astigmatischen Korrekturen gesprochen wird, sind diese lediglich exemplarisch für rotationssymmetrische Refraktionsveränderungen und nichtrotationssymmetrische Refraktionsveränderungen. Auf diese Weise können durchaus Korrekturen höherer Ordnung ausgeführt werden. Weiter ist für den Fall der Überkorrektur diese im Falle eines nicht-rotationssymmetrischen Refraktionskorrekturbedarfs mindestens auf eine Hauptachse der Rotationsasymmetrie bezogen, im Falle des Astigmatismus auf diejenige mit dem geringeren Krümmungsfehler. Damit kann beispielsweise für diese Hauptachse eine Überkorrektur vorliegen, für die um 90° dazu liegende Achse nicht. Idealerweise wird hier der Korrekturbedarf durch das Implantat in einer Achse exakt abgedeckt. Falls dies nicht möglich ist, sollte bevorzugt die Überkorrektur für beide Achsen vorliegen, um die gewünschte Übereinstimmung in der Grundstruktur zwischen Implantat und Lentikel sicherzustellen.

Soweit hier von dem Bestimmen des Implantats die Rede ist, bezieht sich dies auf die Festlegung des additiven Refraktionsveränderungswertes, z.B. umfassend geometrischen Daten des Implantats, also beispielsweise seiner Erstreckungen. In Weiterbildung ist eine vollständige geometrische Beschreibung des vom Implantat eingenommenen Volumens möglich. Falls ein Set an (Standard-)Implantaten bereitgestellt wird, kann sich das Bestimmen des additiven Refraktionsveränderungswertes auch auf das Auswählen eines Implantates aus dem Set beziehen, da für die einzelnen Implantate des Sets der jeweilige additive Refraktionsveränderungswert bekannt ist. Letztlich ist es für die nachfolgenden Abläufe erforderlich, die Refraktionsveränderung des Implantats festzulegen. Dabei können die Bestimmung des Implantats und das Berechnen der Restrefraktionskorrektur auch zusammengefasst werden, indem der Refraktionskorrekturbedarf in eine Refraktionsveränderung, welche durch das Implantat bewirkt wird, und einen verbleibenden Rest zerlegt wird. Dieser Rest ist dann die Restrefraktionskorrektur. Auf diese Weise ist in einem sehr kompakten Ablauf zuerst die Refraktionsveränderung als wesentlicher Parameter für das Implantat festgelegt und damit das Implantat bestimmt worden und zugleich auch die Restrefraktionskorrektur berechnet, für die dann passend das Lentikel und die Schnittfläche, welche das Lentikel umgrenzt, ermittelt werden. Man muss zwischen der Korrektur, die durch Extraktion des Lentikels und Einfügung des Implantats jeweils bewirkt wird, und der Brechkraft des Lentikels bzw. Implantats selbst unterscheiden.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine schematische Darstellung einer Behandlungsvorrichtung mit einer Planungseinrichtung für eine augenchirurgische Refraktionskorrektur,
- Fig. 2: eine schematische Darstellung der Wirkung der Laserstrahlung, die in der Behandlungsvorrichtung der Fig. 1 verwendet wird,
- Fig. 3: eine weitere Schemadarstellung des Behandlungsgerätes der Fig. 1 hinsichtlich der Einbringung der Laserstrahlung,
- Fig. 4: eine schematische Schnittdarstellung durch die Augenhornhaut zur Veranschaulichung der Entnahme des Hornhaut-Volumens im Zusammenhang mit der augenchirurgischen Refraktionskorrektur,
- Fig. 5: eine Schemadarstellung hinsichtlich des Aufbaus des Behandlungsgerätes der Fig. 1 mit besonderem Bezug auf die dort vorhandene Planungseinrichtung,
- Fig. 6: eine Schemadarstellung zur Verdeutlichung eines Zusammenwirkens aus additiver und subtraktiver Fehlsichtigkeitskorrektur,
- Fig. 7: ein Blockschaltbild zu einem Verfahren zur Ermittlung von wesentlichen Daten für das chirurgische Verfahren, beispielsweise der Fig. 6,
- Fig. 8A bis 8C: verschiedene Ansichten der Hornhaut eines Auges zum Erzeugen einer Schnittfläche für die Entnahme eines Lentikels, wobei Fig. 8A eine Draufsicht auf die Hornhaut zeigt und Fig. 8B eine Schnittdarstellung entlang einer vertikalen Achse in Fig. 8A und Fig. 8C entlang einer horizontalen Achse in Fig. 8A widergibt, und
- Fig. 9: eine Schnittdarstellung ähnlich der Fig. 8C nach Einfügen eines Implantats.

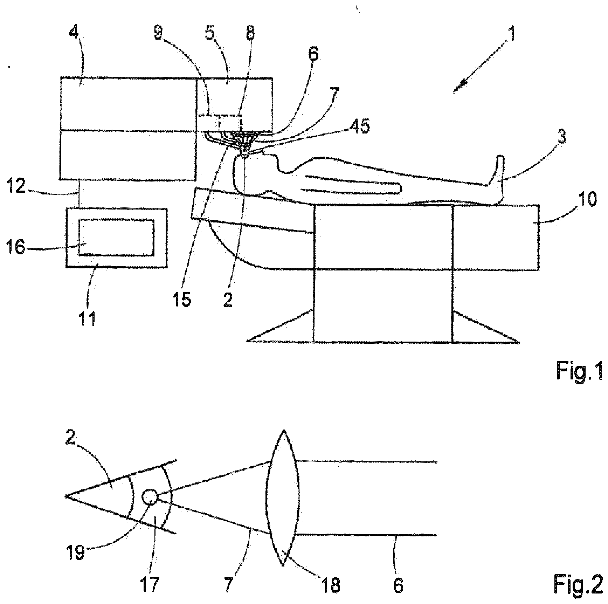

Eine Behandlungsvorrichtung 1 für die Augenchirurgie ist in Fig. 1 dargestellt. Die Behandlungsvorrichtung 1 ist für die Einbringung von Laserschnitten an einem Auge 2 eines Patienten 3 ausgebildet. Dazu weist die Behandlungsvorrichtung 1 eine Lasereinrichtung 4 auf, die aus einer Laserquelle 5 einen Laserstrahl 6 abgibt, welcher als fokussierter Strahl 7 in das Auge 2 bzw. die Augenhornhaut gerichtet wird. Vorzugsweise ist der Laserstrahl 6 ein gepulster Laserstrahl mit einer Wellenlänge zwischen 300 Nanometer und 10 Mikrometer. Weiter liegt die Pulslänge des Laserstrahls 6 im Bereich zwischen 1 Femtosekunde und 100 Nanosekunden, wobei Pulswiederholraten von 50 bis 20000 Kilohertz und Pulsenergien zwischen 0,01 Mikrojoule und 0,01 Millijoule möglich sind. Die Behandlungsvorrichtung 1 erzeugt in der Hornhaut des Auges 2 durch Ablenkung der gepulsten Laserstrahlung eine Schnittfläche. In der Lasereinrichtung 4 bzw. deren Laserquelle 5 sind dazu ein Scanner 8 sowie ein Strahlungsintensitätsmodulator 9 vorgesehen.

Der Patient 3 befindet sich z.B. auf einer Liege 10, die in drei Raumrichtungen verstellbar ist, um das Auge 2 passend zum Einfall des Laserstrahls 6 auszurichten. In bevorzugter Bauweise ist die Liege 10 motorisch verstellbar. Eine Verstellung der Lasereinrichtung 4 ist alternativ möglich. Die Ansteuerung kann insbesondere durch ein Steuergerät 11 erfolgen, das grundsätzlich den Betrieb der Behandlungsvorrichtung 1 steuert und dazu über geeignete Datenverbindungen, beispielsweise Verbindungsleitungen 12 mit der Behandlungsvorrichtung verbunden ist. Natürlich kann diese Kommunikation auch über andere Wege, beispielsweise Lichtleiter oder per Funk geschehen. Das Steuergerät 11 nimmt die entsprechenden Einstellungen, Zeitsteuerung an der Behandlungsvorrichtung 1, insbesondere der Lasereinrichtung 4 vor und bewerkstelligt damit einen entsprechenden Verfahrensablauf an der Behandlungsvorrichtung 1.

Die Behandlungsvorrichtung 1 weist eine Fixiereinrichtung 15 auf, welche die Hornhaut des Auges 2 gegenüber der Lasereinrichtung 4 lagefixiert. Diese Fixiereinrichtung 15 kann ein bekanntes Kontaktglas 45 umfassen, an das die Augenhornhaut durch Unterdruck angelegt wird und das der Augenhornhaut eine gewünschte geometrische Form verleiht. Solche Kontaktgläser sind dem Fachmann aus dem Stand der Technik bekannt, beispielsweise aus der DE 102005040338 A1.

Die Behandlungseinrichtung 1 weist weiter eine nicht dargestellte Kamera auf, welche durch das Kontaktglas 45 hindurch ein Bild der Augenhornhaut 17 aufnehmen kann. Dabei kann die Beleuchtung für die Kamera sowohl im sichtbaren als auch im infraroten Spektralbereich erfolgen.

Das Steuergerät 11 der Behandlungsvorrichtung 1 weist eine Planungseinrichtung 16 auf, die später noch näher erläutert werden wird und die mindestens eine Berechnungseinrichtung umfasst, welche zur Vorbereitung die Schnittfläche(n) und/oder Steuerdaten, insbesondere Ansteuerdaten für die Behandlungsvorrichtung berechnet, damit die die Schnittfläche(n) im chirurgischen Verfahren erzeugt werden kann/können.

Fig. 2 zeigt schematisch die Wirkungsweise des einfallenden Laserstrahls 6. Der Laserstrahl 6 fällt als fokussierter Laserstrahl 7 in die Hornhaut 17 des Auges 2. Zur Fokussierung ist eine schematisch eingezeichnete Optik 18 vorgesehen. Sie bewirkt in der Hornhaut 17 einen Fokus 19, in dem die Laserstrahlungsenergiedichte so hoch ist, dass in Kombination mit der Pulslänge der gepulsten Laserstrahlung 6 ein nicht-linearer Effekt in der Hornhaut 17 auftritt. Beispielsweise kann jeder Puls der gepulsten Laserstrahlung 6 im Fokus 19 einen optischen Durchbruch in der Augenhornhaut 17 erzeugen, welche wiederum eine in Fig. 2 nur schematisch angedeutete Plasmablase initiiert. Bei Entstehung der Plasmablase umfasst die Gewebsschichttrennung ein größeres Gebiet als den Fokus 19, obwohl die Bedingungen zur Erzeugung des optischen Durchbruches nur im Fokus 19 erreicht werden. Damit von jedem Laserpuls ein optischer Durchbruch erzeugt wird, muss die Energiedichte, d.h. die Fluence der Laserstrahlung oberhalb eines gewissen, pulslängenabhängigen Schwellwertes liegen. Dieser Zusammenhang ist dem Fachmann beispielsweise aus der DE 69500997 T2 bekannt. Alternativ kann ein gewebetrennender Effekt auch durch gepulste Laserstrahlung erreicht werden, indem mehrere Laserstrahlungspulse in einem Bereich abgegeben werden, wobei sich die Fokus-Spots überlappen. Es wirken dann mehrere Laserstrahlungspulse zusammen, um einen gewebetrennenden Effekt zu erreichen. Die Art der Gewebetrennung, die die Behandlungsvorrichtung 1 einsetzt, ist jedoch für die nachfolgende Beschreibung nicht weiter relevant; wesentlich ist lediglich, dass eine Schnittflächenerzeugung in der Hornhaut 17 des Auges 2 stattfindet.

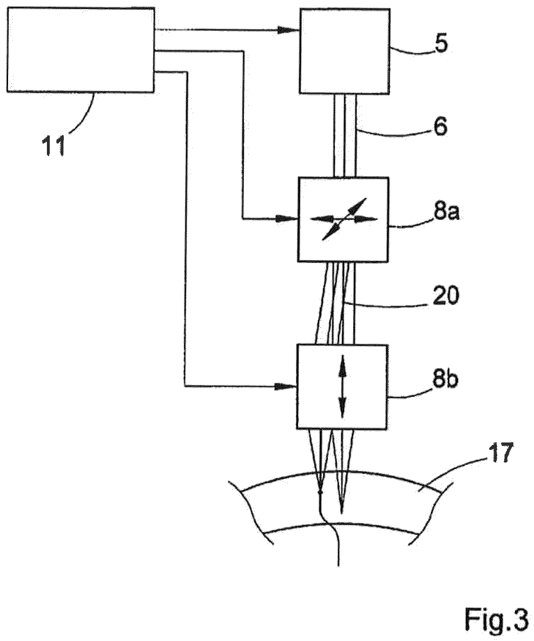

Um eine augenchirurgische Refraktionskorrektur auszuführen, wird mittels der Laserstrahlung 6 aus einem Gebiet innerhalb der Hornhaut 17 ein als Lentikel bezeichnetes Hornhautvolumen entfernt, indem innerhalb der Hornhaut Gewebeschichten getrennt werden, die das Hornhaut-Volumen isolieren und dann dessen Entnahme ermöglichen. Das Hornhaut-Volumen wird durch eine dreidimensional geformte Schnittfläche umgrenzt. Hierzu wird im Falle der gepulst eingebrachten Laserstrahlung die Lage des Fokus 17 der fokussierten Laserstrahlung 7 dreidimensional in der Hornhaut 17 verstellt. Dies ist schematisch in Fig. 3 gezeigt. Die Krümmung der Vorderseite der Hornhaut 17 wird durch die Entnahme des Lentikels gezielt verändert, um so die Refraktionskorrektur zu erreichen. Ein gleichmäßig dickes Volumen würde die Krümmung der Vorderseite der Hornhaut nicht wesentlich ändern - daher der Begriff Lentikel.

In Fig. 3 sind die Elemente der Behandlungsvorrichtung 1 nur insoweit eingetragen, als sie zum Verständnis der Schnittflächenerzeugung erforderlich sind. Der Laserstrahl 6 wird, wie bereits erwähnt, in einen Fokus 19 in der Hornhaut 19 gebündelt, und die Lage des Fokus 19 in der Hornhaut wird verstellt, so dass zur Schnittflächenerzeugung an verschiedenen Stellen fokussierende Energie aus Laserstrahlungspulsen in das Gewebe der Hornhaut 17 eingetragen wird. Die Laserstrahlung 6 wird von der Laserquelle 5 vorzugsweise als gepulste Strahlung bereitgestellt. Der Scanner 8 ist in der Bauweise der Fig. 3 zweiteilig aufgebaut und besteht aus einem xy-Scanner 8a, der in einer Variante durch zwei im Wesentlichen orthogonal ablenkende Galvanometerspiegel realisiert ist. Der Scanner 8a lenkt den von der Laserquelle 5 kommenden Laserstrahl 6 zweidimensional ab, so dass nach dem Scanner 9 ein abgelenkter Laserstrahl 20 vorliegt. Der Scanner 8a bewirkt somit eine Verstellung der Lage des Fokus 19 im Wesentlichen senkrecht zur Haupteinfallsrichtung des Laserstrahls 6 in der Hornhaut 17. Zur Verstellung der Tiefenlage ist neben dem xy-Scanner 8a im Scanner 8 noch ein z-Scanner 8b vorgesehen, der beispielsweise als verstellbares Teleskop ausgebildet ist. Der z-Scanner 8b sorgt dafür, dass die z-Position der Lage des Fokus 19, d.h. dessen Position auf der optischen Achse des Einfalls verändert wird. Der z-Scanner 8b kann dem xy-Scanner 8a nach- oder vorgeordnet sein. Die Scann verschieben z.B. den Fokus 19 längs einer dreidimensionalen Bahn, entlang der die Laserpulse abgegeben werden, um die Schnittfläche(n) auszubilden.

Für das Funktionsprinzip der Behandlungsvorrichtung 1 ist die Zuordnung der einzelnen Koordinaten zu dem Raumrichtungen nicht wesentlich, genau so wenig, dass der Scanner 8a um zueinander rechtwinklige Achsen ablenkt. Vielmehr kann jeder Scanner verwendet werden, der in der Lage ist, den Fokus 19 in einer Ebene zu verstellen, in der die Einfallsachse der optischen Strahlung nicht liegt. Auch können beliebige nicht-kartesische Koordinatensystem zur Ablenkung bzw. Steuerung der Lage des Fokus 19 verwendet werden. Beispiele dafür sind Kugelkoordinaten oder zylindrische Koordinaten. Die Steuerung der Lage des Fokus 19 erfolgt mittels der Scanner 8a, 8b unter Ansteuerung durch das Steuergerät 11, das entsprechende Einstellungen an der Laserquelle 5, dem (in Fig. 3 nicht gezeigten) Modulator 9 sowie dem Scanner 8 vornimmt. Das Steuergerät 11 sorgt für einen geeigneten Betrieb der Laserquelle 5 sowie die hier exemplarisch geschilderte dreidimensionale Fokusverstellung, so dass letztendlich eine Schnittfläche ausgebildet wird, die ein bestimmtes Hornhaut-Volumen isoliert, das zur Refraktionskorrektur entfernt bzw. entnommen werden soll. Es arbeitet dabei nach ihm vorgegebenen Ansteuerdaten.

Die Ansteuerdaten sind beispielsweise als Zielpunkte für die Fokusverstellung und/oder als Daten für die genannte Bahn vorgegeben. Die Ansteuerdaten sind in der Regel in einem Ansteuerdatensatz zusammengefasst. Dieser gibt geometrische Vorgaben für die auszubildende Schnittfläche, beispielsweise die Koordinaten der Zielpunkte als Muster vor. Der Ansteuerdatensatz enthält dann in dieser Ausführungsform auch konkrete Stellwerte für den Fokuslagenverstellmechanismus, z.B. für den Scanner 8. Die Ansteuerdaten beruhen auf Steuerdaten, welche die zu erzeugende(n) Schnittfläche(n) spezifizieren, wie es noch erläutert werden wird.

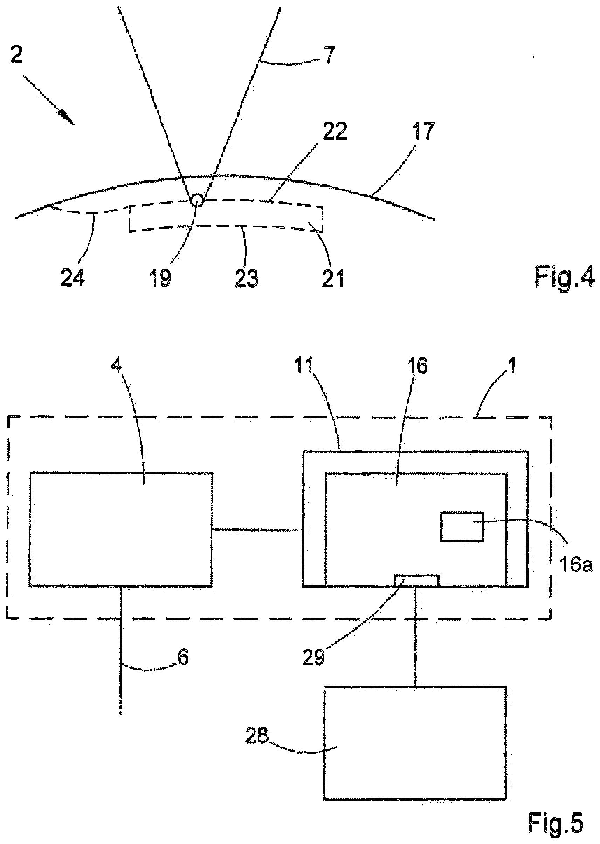

Die Erzeugung der Schnittfläche mit der Behandlungsvorrichtung 1 ist exemplarisch in Fig. 4 gezeigt. Ein Hornhaut-Volumen 21 in der Hornhaut 17 wird durch Verstellung des Fokus 19, in den der fokussierte Strahl 7 gebündelt ist, isoliert. Es wird eine mehrteilige Schnittfläche ausgebildet, weshalb auch von "Schnittfläche(n)" die Rede ist. Sie weist hier exemplarisch eine anteriore Flap-Schnittfläche 22 sowie als posteriore Lentikel-Schnittfläche 23 auf. Diese Begriffe sind hier lediglich exemplarisch zu verstehen und sollen nur den Bezug auf das herkömmliche Lasik- oder Flex-Verfahren herstellen, für das die Behandlungsvorrichtung 1 optional ebenfalls ausgebildet ist. Wesentlich ist hier lediglich, dass die Schnittflächen 22 und 23 sowie ggf. nicht weiter bezeichnete Randschnitte, welche die Schnittflächen 22 und 23 an deren Rändern zusammenführen, das Hornhaut-Volumen 21 umgrenzen und isolieren. Durch einen Öffnungsschnitt 24 kann das Hornhaut-Volumen 21 entnommen werden, wie es SMILE-Verfahren gemäß DE 102007019813 A1 vorsieht.

Fig. 5 zeigt schematisch die Behandlungsvorrichtung 1, und anhand ihr soll die Bedeutung der Planungseinrichtung 16 näher erläutert werden. Die Behandlungsvorrichtung 1 weist in dieser Variante mindestens zwei Einrichtungen oder Module auf. Die bereits geschilderte Lasereinrichtung 4 gibt den Laserstrahl 6 auf das Auge 2 ab. Der Betrieb der Lasereinrichtung 4 erfolgt dabei, wie bereits geschildert, voll automatisch durch das Steuergerät 11, d.h. die Lasereinrichtung 4 startet auf ein entsprechendes Startsignal hin die Erzeugung und Ablenkung des Laserstrahls 6 und erzeugt Schnittflächen, die auf die beschriebene Art und Weise aufgebaut sind. Die für den Betrieb erforderlichen Steuersignale empfängt die Lasereinrichtung 5 vom Steuergerät 11, dem zuvor entsprechende Steuerdaten bereitgestellt wurden. Dies erfolgt z. B. mittels der Planungseinrichtung 16, die in Fig. 5 lediglich exemplarisch als Bestandteil des Steuergeräts 11 gezeigt ist. Natürlich kann die Planungseinrichtung 16 auch eigenständig ausgebildet sein und drahtgebunden oder drahtlos mit der Steuereinrichtung 11 kommunizieren. Wesentlich ist dann lediglich, dass ein entsprechender Datenübertragungskanal zwischen der Planungseinrichtung 16 und dem Steuergerät 11 vorgesehen ist.

Die Planungseinrichtung 16 umfasst als Kernelement eine Berechnungseinrichtung 16a, welche, wie nachfolgend noch erläutert, die Schnittfläche(n) berechnet, die in der Hornhaut 17 erzeugt werden sollen, und die für diese Schnittflächen nötigen Daten ermittelt. Die Planungseinrichtung 16 oder direkt die Berechnungseinrichtung 16a erzeugt daraus den Ansteuerdatensatz, der dem Steuergerät 11 zur Ausführung der augenchirurgischen Refraktionskorrektur zur Verfügung gestellt wird.

Zur Berechnung der Schnittflächen(n) verwendet die Berechnungseinrichtung 16a Messdaten über die Hornhaut des Auges. Diese Messdaten stammen in der hier beschriebenen Ausführungsform aus einer Messeinrichtung 28, die das Auge 2 des Patienten 2 zuvor vermessen hat. Natürlich kann die Messeinrichtung 28 auf beliebige Art und Weise ausgebildet sein und die entsprechenden Messdaten an die Schnittstelle 29 der Planungseinrichtung 16 übermitteln.

Die Planungseinrichtung unterstützt den Bediener der Behandlungsvorrichtung 1 bei der Festlegung der Schnittfläche zur Isolierung des Hornhaut-Volumens 21. Dies kann bis zu einer vollautomatischen Festlegung der Schnittflächen gehen, die beispielsweise dadurch bewirkt werden kann, dass die Berechnungseinrichtung 16a aus den Messdaten das zu entnehmende Hornhaut-Volumen 21 ermittelt, dessen Begrenzungsfläche(n) als Schnittfläche(n) definiert und daraus entsprechende Steuerdaten für das Steuergerät 11 erzeugt. Am anderen Ende des Automatisierungsgrades kann die Planungseinrichtung 16 Eingabemöglichkeiten vorsehen, an denen ein Benutzer die Schnittflächen in Form von geometrischen Parametern etc. eingibt. Zwischenstufen sehen Vorschläge für die Schnittflächen vor, welche die Planungseinrichtung 16 automatisch generiert und die von einem Bearbeiter dann modifizierbar sind. Grundsätzlich können all diejenigen Konzepte, die im vorstehend allgemeineren Beschreibungsteil bereits erläutert wurden, hier in der Berechnungseinrichtung 16a zur Anwendung kommen.

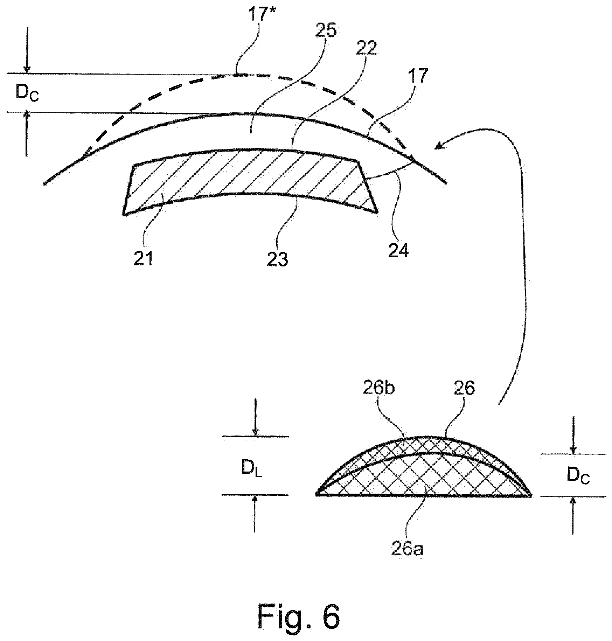

Fig. 6 zeigt das zugrundeliegende Prinzip der Fehlsichtigkeitskorrektur, für welches die Berechnungseinrichtung 16a die wesentlichen Daten bereitstellt und welches mittels der Behandlungsvorrichtung 1 ausgeführt werden kann. Fig. 6 zeigt dabei eine Schnittdarstellung ähnlich der Fig. 4, und übereinstimmende Elemente sind mit denselben Bezugszeichen wie in Fig. 4 bezeichnet. Der obere Teil der Fig. 6 zeigt die Hornhaut 17 des Auges, wobei gestrichelt die Hornhaut mit veränderter Form eingezeichnet ist, die sich nach dem chirurgischen Eingriff einstellt und mit 17* bezeichnet ist. Wie zu sehen ist, hat nach dem chirurgischen Eingriff die Vorderseite der Hornhaut 17* eine stärkere Krümmung. In die nicht maßstäbliche Zeichnung ist dabei eine Krümmungserhöhung in der Mitte mit einem Maß D_{C} eingetragen. In der Hornhaut wird mittels der genannten Schnittflächen 22, 23, die zusammen eine dreidimensionale Schnittfläche bilden, das Lentikel 21 isoliert. Es sei darauf hingewiesen, dass in der dargestellten Ausführungsform dieses Lentikel 21 nicht die Krümmungserhöhung bewirken würde, sondern eigentlich die Krümmung abflachen würde. Hierauf wird später noch eingegangen. Nachdem das Lentikel 21 isoliert und durch den Öffnungsschnitt 24 entnommen wurde, wird ein Implantat 26 eingegliedert, das in Fig. 6 schematisch rechts unten dargestellt ist. Der Eingliederungsvorgang ist durch einen Pfeil symbolisiert, der anzeigen soll, dass das Lentikel 26 durch den Öffnungsschnitt 24 in die Kammer eingeführt wird, die sich nach dem Entnehmen des Lentikels 21 gebildet hat und welche durch die Flächen 22, 23 begrenzt ist. Fig. 6 zeigt, dass das Lentikel 21 eine Höhe D_{L} aufweist, die größer ist, als die Höhe D_{C}, um welche die Krümmung der Hornhaut 17* nach dem chirurgischen Eingriff größer sein soll. Der Unterschied zwischen D_{L} und D_{C} kann in einer vereinfachten Erläuterung damit erklärt werden, dass das Lentikel 26 aus zwei Bestandteilen 26a und 26b zusammengesetzt angenommen werden kann. Der Bestandteil 26a entspricht letztlich der Krümmungsänderung für die Hornhaut 17* nach dem Eingriff. Er hat damit in dieser vereinfachenden Erläuterung die Höhe D_{C}. Der restliche Teil 26b, in den das Implantat 26 gedanklich unterteilt werden kann, entspricht dem Lentikel 21. Auf diese Weise kann ein Lentikel 21 verwendet werden, das an und für sich für den gewünschten Effekt - hier eine Erhöhung der Krümmung der Hornhaut 17 - gar nicht geeignet wäre. Durch diese Abstimmung wird erreicht, dass das Lentikel 21 und das Implantat 26 die gleiche Grundform haben, nämlich eine höhere Dicke im Zentrum als am Rand.

Fig. 6 zeigt hierbei eine Vereinfachung. Diese beruht darauf, dass für die Änderung der Krümmung der Augenhornhaut der Unterschied zwischen der Unterseite des Lentikels/Implantats und der Oberseite des Lentikels/Implantats relevant ist, wobei es auf den Unterschied in der Krümmung dieser beiden Seiten ankommt. Die absolute Dicke von Lentikel/Implantat spielt hingegen für die Refraktionsänderung, d.h. die Änderung der Krümmung der Hornhautvorderseite keine wesentliche Rolle. Dies erkennt man beispielsweise daran, dass das Einfügen oder Entnehmen eines Volumens, welches über seine laterale Erstreckung eine konstante Dicke hat, im eingefügten Bereich die Krümmung der Hornhautvorderseite nur sehr wenig ändern würde, sondern lediglich dafür sorgen würde, dass die Hornhautvorderseite etwas anteriorer oder posteriorer liegt, aber annähernd die gleiche Krümmung behält. Fig. 6 zeigt deshalb sehr schematisch das Lentikel 21 mit einer größeren Randdicke, da es hierdurch erreicht wird, dass das Volumen des entnommenen Lentikels 21 größer ist, als das einzufügende Implantat 26. Auf die Vorteile dieser Option, die nicht zwingend ist, wird nachfolgend noch eingegangen. Die schematische Darstellung der Fig. 6 darf jedoch nicht damit verbunden werden, dass die Randdicke des Lentikels 21 in einem Maß, das zur Refraktionsveränderung ausreichen würde, zur Änderung der Krümmung der Hornhautvorderseite beitragen würde. Dies ist nicht der Fall. Für die Refraktionskorrektur sind vielmehr bezogen auf das Lentikel 21 hauptsächlich die Krümmungsunterschiede zwischen den Flächen 22 und 23 relevant, und diese Krümmungsunterschiede sind auch in der schematischen Darstellung des Lentikels 26 für den Teil 26b eingetragen. Diese Vereinfachung darf nicht damit verwechselt werden, dass das Lentikel 21 exakt in der Form des Teiles 26b ausgeführt wird, was die Gesamtdicke angeht. Eine derartige Übereinstimmung ist zwar möglich, aber nicht zwingend erforderlich.

Die Abstimmung von Lentikel 21 und Implantat 26 wird dadurch erreicht, dass die Berechnungseinrichtung 16a zuerst den additiven Refraktionsveränderungswert für das Implantat 26 definiert und dann das Lentikel 21 hinsichtlich dessen subtraktiven Refraktionsveränderungswert so abstimmt, dass insgesamt die gewünschte Krümmungsänderung der Hornhaut 17* nach dem Eingriff erreicht ist. Dabei ist im Ausführungsbeispiel der Fig. 6 das Implantat 26 so gewählt worden, dass es eine Überkorrektur ausführt. Mit anderen Worten, das Implantat 26 alleine würde durch den additiven Refraktionsveränderungswert eine zu große Krümmungserhöhung bewirken. Im dargestellten Beispiel im Fall der Fig. 6 hätte das zur Folge, dass eine bestehende Hyperopie (Hornhaut ist zu flach) in eine Myopie (Hornhaut ist zu steil) umgewandelt würde. Dies tritt dennoch nicht ein, da die Berechnungseinrichtung 16a nach der entsprechenden Wahl eines überkorrigierenden Implantats 26 den subtraktiven Refraktionsveränderungswert für das Lentikel 21 und damit die Schnittflächen 22, 23, welche das Lentikel 21 definieren, so festlegt, dass insgesamt genau die gewünschte Korrektur erreicht wird. Dies ist insgesamt dadurch möglich, dass zwei Gesichtspunkte kombiniert werden. Zum einen wird die additive Fehlsichtigkeitskorrektur (Einfügen des Implantats 26) mit der subtraktiven Fehlsichtigkeitskorrektur (Entfernen bzw. Entnahme des Lentikels 21) kombiniert. Diese Kombination erfolgt zudem zielgerichtet und geht von der Festlegung des Implantats 26 aus und passt das Lentikel 21 daran an.

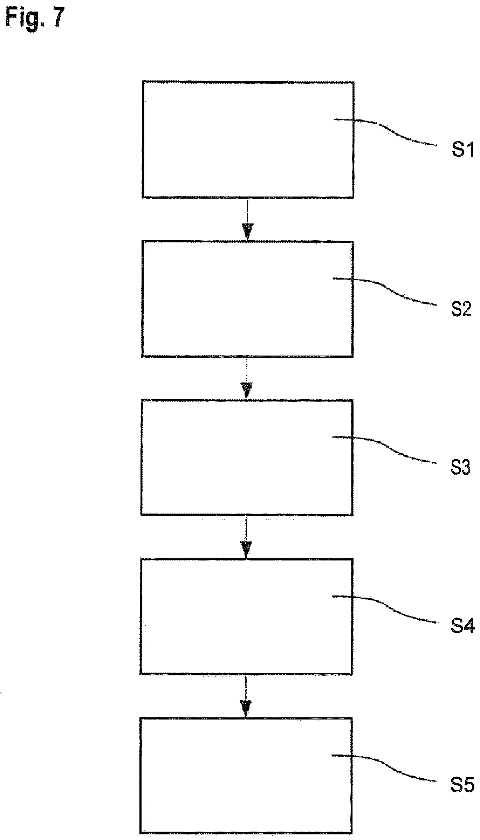

Fig. 7 zeigt ein Blockschaltbild für ein entsprechendes Verfahren zum Ermitteln der wesentlichen Daten. In einem Schritt S1 werden Daten über einen Refraktionskorrekturbedarf des Auges empfangen, die beispielsweise in einem vorgelagerten Schritt bereitgestellt werden können, was insbesondere eine Vermessung des Auges umfassen kann. Auf Basis dieses Refraktionskorrekturbedarfs wird in einem Schritt S2 die wesentliche Größe für ein einzufügendes Implantat festlegt und dadurch das Implantat bestimmt. Diese Größe ist der vom Implantat bewirkte additive Refraktionsveränderungswert.

In einem Schritt S3 wird ausschließlich der subtraktive Refraktionsveränderungswert berechnet, der sich aufgrund des Refraktionskorrekturbedarfs und der additiven Refraktionsveränderung, welche das Implantat bewirken wird, nötig ist. Die Schritte S2 und S3 können dabei, wie im allgemeinen Teil der Beschreibung erläutert und später auch noch weiter dargelegt, kombiniert werden. In einem anschließenden Schritt S4 wird das in der Hornhaut zu isolierende Lentikel auf Basis des subtraktiven Refraktionsveränderungswerts berechnet. Dabei wird das Lentikel so ausgelegt, dass nach seiner Entnahme aus der Hornhaut die nötige, ergänzende Restrefraktionskorrektur bewirkt ist. Damit haben Implantat und Lentikel in Zusammenwirkung genau diejenige Hornhautmodifikation zur Folge, die zur Abdeckung des Refraktionskorrekturbedarfs erforderlich ist. Hierbei kann die erwähnte Volumenabstimmung bewirkt werden.

In einem abschließenden Schritt S5 wird die Schnittfläche berechnet, die erforderlich ist, um das Lentikel in der Hornhaut zu isolieren. Diese das Lentikel isolierende Schnittfläche (z.B. 22, 23 und eine Randfläche zum Einstellen der Gesamtdicke) definiert zugleich eine Kammer für das einzusetzende Implantat. Für diese Schnittfläche werden entsprechende Daten erzeugt, welche die Schnittfläche beschreiben. Diese Daten dienen dann als Steuerdaten zur Korrektur der Refraktion des Auges, wobei es sich durchaus noch um Rohdaten handeln kann, die erst in entsprechende Ansteuerungsdaten für die Behandlungsvorrichtung überführt werden müssen, beispielsweise durch Definition der zuvor bereits erwähnten Bahnen, entlang welcher der Fokus verstellt wird.

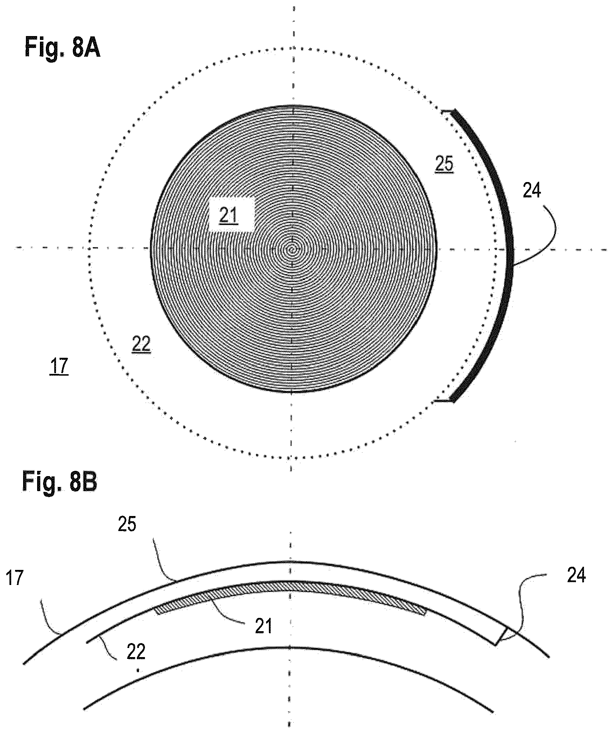

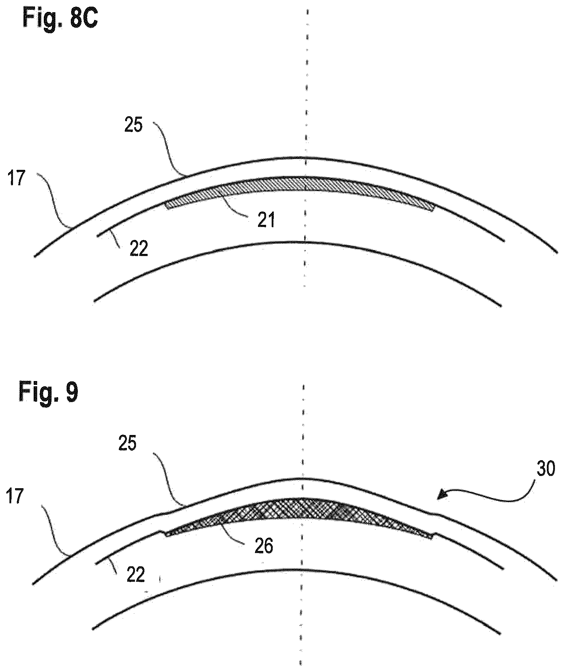

Fig. 8A zeigt eine Draufsicht auf die Hornhaut 17 des Auges für eine Ausführungsform, bei der eine astigmatische Korrektur vorgenommen werden soll. Die darunter gezeichnete Schnittdarstellung der Fig. 8B zeigt den Schnitt in der Horizontalachse durch die Fig. 8A. Hier ist das zu entnehmende Lentikel 21 eingezeichnet. Wie der Vergleich mit der Fig. 8C zeigt, welche die Schnittdarstellung durch die Vertikalachse der Fig. 8A darstellt, ist das Lentikel 21 nicht rotationssymmetrisch, da es in den beiden Schnitten unterschiedliche Krümmungen hat. Es ist in seiner Grundstruktur, wie auch in Fig. 6, so ausgebildet, dass es die Krümmung der Hornhautvorderseite reduzieren würde. Dennoch wird insgesamt eine Hyperopiekorrektur mit einer Erhöhung der Krümmung der Hornhautvorderseite durchgeführt. Dies zeigt Fig. 9, die eine Schnittdarstellung ähnlich der Fig. 8A zeigt, hier jedoch nach Einfügung des Implantats 26, welches kreuzschraffiert gezeichnet ist. Das Implantat 26 hat eine Grundform, welche der dem entnommen Lentikel gleicht, das, wie Fig. 8C gut erkennen lässt, in der Mittel dicker ist, als am Rand. Es wird damit das Prinzip der Fig. 6 auch hier verfolgt, allerdings im Falle einer astigmatischen Korrektur. Bei dieser ist das Implantat 26 rotationssymmetrisch, d.h. die Darstellung der Fig. 9 gilt gleichermaßen (bis auf den Öffnungsschnitt 24) auch für die horizontale Schnittebene der Fig. 8A - zumindest was die Ausgestaltung des Implantats 26 angeht, denn die Kammer, welche durch die Entnahme des Lentikels 21 geschaffen wurde, ist nicht rotationssymmetrisch.

Fig. 9 zeigt, dass in einer Randzone 30, die bei Hyperopiekorrekturen mit dem SMILE-Prinzip regelmäßig problematisch ist, kein Problem entsteht, da die Grundform von Lentikel 21 und Implantat 6 dieselbe ist, so dass an der Randzone 30, an der die überliegende Membran 25 mit dem Rest der Hornhaut 17 zusammenkommt, keine großen Spannungen oder Stufen entstehen.

Die Verwendung eines rotationssymmetrischen Implantats 26 hat weiter den Vorteil, dass dessen Rotationsstellung, d.h. die Drehlage um die strichpunktierte Achse der Fig. 9, bei der Einfügung des Implantats 26 völlig irrelevant ist, denn die Rotationsasymmetrie, d.h. der Astigmatismus wird durch die rotationsasymmetrische Kammer, geschaffen durch die Entnahme eines rotationsasymmetrischen Lentikels 21, erreicht.

Das Vorgehen, im Verfahren im Schritt S2 mit der Bestimmung des Implantats zu beginnen und darauf ausgerichtet die durch das Lentikel abzudeckende Refraktionskorrektur zu bestimmen hat weiter den Vorteil, dass mit einem Satz an Implantaten gearbeitet werden kann, die Standardgrößen haben. Es ist keine individuelle exakte Anpassung des Implantats 26 an den konkreten Refraktionskorrekturbedarf nötig, da dies im Schritt S3 durch die Bestimmung des subtraktiven Refraktionsveränderungswertes erreicht wird. Ein beispielhaftes Vorgehen für die Verwendung eines solchen Satzes an Standardimplantaten 26 wird nachfolgend am Beispiel einer Hyperopie-Korrektur (S_{T}<0) erläutert:
1. Eingabe des manifesten, also prä-operativen Refraktionsfehlers eines Auges eines Patienten.
2. Eingabe der Zielrefraktion (auch evtl. höhere Ordnungen).
3. Berechnung der angestrebten refraktiven Korrektur B_{T} als Differenz; dies ist der Refraktionskorrekturbedarf. Beträgt z.B. die post-operative Zielrefraktion (Restfehlsichtigkeit) 0 dpt und ist der prä-operative Fehler +5 dpt (also eine Hyperopie), so strebt man eine Korrektur B_{T} = -5 dpt an. Eine Hyperopiekorrektur erfordert also eine Korrektur um einen negativen Dioptrienwert, auch wenn der prä-operative Sehfehler durch eine positive Dioptrienangabe beschrieben wird.
4. Zerlegung der Korrektur in Sphäre, Zylinder, höhere Ordnungen (S_{T}, C_{T}, X_{T}).
5. Vergleich der Sphäre S_{T} mit einem Set Sᵢ : Auswahl der additiven Korrekturstärke durch Identifizieren des (positiv oder negativ) benachbarten Elements aus dem Set mit Sᵢ > -S_{T} (prinzipiell ist jedes Sᵢ geeignet, aber der nächstgelegene Nachbar ist bevorzugt). Bleibt man beim unter 3. genannten Beispiel, wird man aus einem Set von Implantaten, das für geradzahlige Dioptrienwerte vorliegt, das Implantat mit Sᵢ = +6 dpt auswählen, denn es gilt B_{T} = -5 dpt = 1 dpt - 6 dpt. Die Sᵢ sind die (sphärische) Brechkraftwerte des Implantats 26. Dadurch ist der additive Refraktionsveränderungswert bestimmt. Eine Myopie-SMILE entnimmt dann ein positiv brechendes Lentikel 21 mit den noch fehlenden subtraktiven Refraktionsänderungswerten B_{S}. Die Berechnung der subtraktiv zu erzielenden Brechkraftdifferenz B_{S} erfolgt aus der Korrektur B_{T} und der gewählten additiven Korrekturstärke Sᵢ zum Bereitstellen der angestrebten Korrektur durch eine subtraktive und eine additive Komponente: B_{T} = B_{S} - Sᵢ
6. Berechnung der Lentikelform zur Korrektur B_{S}. B_{S} beschreibt die Brechkraft des entnommenen Lentikels.
7. Erzeugung von Daten, welche eine Schnittfläche beschreiben, die das Lentikel 21 umgrenzt, und Erzeugen von Steuerdaten zur Erzeugung der Schnittfläche in der berechneten Form.

Für einen Patienten wird dasjenige Implantat 26 aus dem Set ausgewählt, das in Kombination mit der Entfernung bzw. Entnahme eines für den Refraktionskorrekturbedarf geeignet berechneten Lentikels den gewünschten Effekt erzielt, wobei die Kombination zu einer materialschonenden OP führt.

Grundsätzlich kann die Brechkraft B_{S} des entnommenen Lentikels positiv oder negativ sein. Das entspricht im ersteren Fall einer Myopiekorrektur (Brechkraft des entfernten bzw. entnommenen Lentikels ist positiv) und im zweiten Fall einer Hyperopiekorrektur für sich, wobei im Folgenden auf die Variante der Hyperopiekorrektur im Detail eingegangen wird. Insbesondere höhere Ordnungen, aber auch sphärische Komponenten sind möglich, echt gemischte Varianten ebenso. Aus applikativer Sicht ist es bei Hyperopiekorrektur bevorzugt, die subtraktive Komponente vollständig in den Bereich der SMILE-Myopie zu legen, also dafür zu sorgen, dass die Entnahme des Lentikels 21 in beiden Haupt-Meridianen eine negative Brechkraftkorrektur bewirkt. Auf diese Weise hat das Lentikel in beiden Meridianen eine positive Brechkraft und damit eine vorteilhafte physiologische Form (Mitte dick, Rand dünn). Das ist aber nicht zwingend notwendig, lediglich eine vorteilhafte Ausführung. Damit dies erreicht wird, wird durch das Implantat 26 die Hyperopie überkorrigiert und dann subtraktiv (zumindest) in der Zylinderkomponente eine Myopiekorrektur durchgeführt. Dieses auf den ersten Blick abwegige Vorgehen sorgt dafür, dass das Implantat auf eine rein sphärische Hyperopie konzentriert wird. Dabei tritt der bereits genannte Vorteil zutage, dass nicht auf eine besondere Orientierung des Implantats geachtet werden muss, weil es keine winkelabhängige Brechkraftkomponente hat. Das obige Verfahren wird dann in folgender Weise modifiziert:
1. Eingabe des manifesten Refraktionsfehlers eines Auges eines Patienten
2. Eingabe der Zielrefraktion (ggf. auch höhere Ordnungen, Multifokalität)
3. Berechnung der angestrebten refraktiven Korrektur B_{T} als Differenz (B_{T} = Target = Refraktion)
4. Zerlegung der Korrektur in Sphäre S_{T_Max}, Zylinder, höhere Ordnungen (S_{T}, C_{T}, X_{T})
5. Berechnung der maximalen B_{T_Max} und minimalen Brechkraft B_{T_Max} in den entsprechenden Meridianen
6. Vergleich der Sphäre S_{T_Max} mit dem Set Sᵢ : Auswahl der additiven Korrekturstärke durch Identifizieren des (positiv oder negativ) benachbarten Elements aus dem Set mit Sᵢ > -S_{T_Max} (prinzipiell ist jedes Sᵢ geeignet, aber der nächstgelegene Nachbar ist bevorzugt). Berechnung der subtraktiv zu erzielenden meridialen Brechkraftdifferenzen B_{S_Min} und B_{S_Min} aus Korrektur B_{T} und gewählter additiver Korrekturstärke Sᵢ zur Zerlegung der angestrebten Korrektur in eine subtraktive und eine additive Komponente B_{T_Max} = B_{S_Max} - Sᵢ und B_{T_Min} = B_{S_Min} - Sᵢ
7. Berechnung der Lentikelform zur Korrektur Bs (mit B_{S_Max} und B_{S_Min})
8. Erzeugung von Steuerdaten zur Erzeugung eines Lentikel der berechneten Form.

Korrekturen höherer Ordnung werden dabei vorzugsweise rein über den subtraktiven Anteil abgedeckt, während die Korrektur der jeweils angepassten Sphäre durch das verfügbare Implantat 26 aus dem Set erfolgt. Ein Vorteil des kombinierten additiv-subtraktiven Korrekturverfahrens ist es also auch, dass hierdurch die Anzahl der notwendigen Implantat-Varianten verringert wird, was Aufwand bei Fertigung und Logistik verringert. Die Anpassung eines Implantats 26 durch den Anwender ist nicht notwendig.

Ein dritter Vorteil ergibt sich überraschend dahingehend, dass bei der Implantation in die Hornhautkammer, die nach vorheriger Lentikelextraktion entsteht, die Elastizität der als Cap 25 darüber liegenden Hornhaut 17 nun ausreicht, um sich der Form des Implantats 26 vollständig anzupassen, da aufgrund der Abmessungen des Lentikels 21 für das Implantat 26 keine große laterale Dehnung mehr erforderlich ist. Hierzu sei auf den Artikel von Gatinel et al. (Gatinel D., Weyhausen A., Bischoff M.; Journal of Refractive Surgery. 2020; 36(12):844-850) verwiesen. Das subtraktive Verfahren schafft den für das Implantat 26 benötigten Raum. Die von Gatinel et al. angegebene Formel zur vereinfachten Berechnung des Volumens einer SMILE-Behandlung der Myopie kann abgewandelt werden, um nun das für das Implantat 26 benötigte Volumen zu berechnen. Gleichung 4 des Artikels lautet: ${V}_{L} \approx 0 , 5 {mm}^{3} - 0 , 28 \frac{{mm}^{3}}{dpt} S - 0 , 41 \frac{{mm}^{3}}{dpt} {C}^{-}$

Diese Gleichung gilt in der negativen Zylindernotation und mit korrekturbezogener Brechkraftangabe (Vorzeichenwechsel) mit den publizierten Konstanten für eine minimale Lentikeldicke (am Rand) von 15 µm, mit welcher der absolute Term skaliert. Zudem gilt die Gleichung für Durchmesser der optischen Zone von 6,5 mm. Für andere Parameter sind für den Fachmann entsprechend andere Konstanten berechenbar.

Allgemein kann man für das subtraktive Volumen schreiben (wobei S_{S} und C_{S} die sphärische bzw. Zylinderkomponente der positiven Brechkraft des für die Korrektur der Myopie zu entfernenden bzw. zu entnehmenden Lentikels bezeichnen.) ${V}_{S} \approx {a}_{S} + b ⋅ {S}_{S} + c ⋅ {{C}_{S}}^{-}$ und für das rein sphärische additive Volumen ${V}_{A} \approx {a}_{A} + b ⋅ {S}_{A}$

Als Vorzeichen gelten Brechkräfte des Lentikels bzw. des Implantats. Mit der Anforderung, dass durch die Lentikelextraktion das Volumen für das Implantat 26 erzeugt werden soll und die Durchmesser der optischen Zonen übereinstimmen, sollte also die folgende Ungleichung gelten: ${V}_{A} \leq {V}_{S}$

Es ist alternativ möglich, die Anforderung zu formulieren, dass die inneren Bogenlängen des Caps 25 (für alle Winkel) größer gleich der äußeren Bogenlängen des Implantats 26 sind, aber die hier formulierte Volumenbedingung kann als Näherung dienen und ist relativ einfach zu berechnen. Setzt man nun die obigen Gleichungen ineinander ein, so ergibt sich daraus: ${a}_{A} + b ⋅ {S}_{A} \leq {a}_{S} + b ⋅ {S}_{S} + c ⋅ {{C}_{S}}^{-}$

Somit folgt als Bedingung für die additive Sphäre: ${S}_{A} \leq \frac{{a}_{S} - {a}_{A}}{b} + {S}_{S} + \frac{c}{b} {{C}_{S}}^{-}$

Diese Bedingung ist am ehesten erfüllbar, wenn eine möglichst große Randdicke beim Lentikel 21 und eine möglichst geringe Randdicke beim Implantat 26 einen möglichst großen ersten Term verursachen. Beispielsweise kann man ein Lentikel 21 mit 30 µm Randdicke entnehmen und ein Implantat 26 mit 15 µm Randdicke implantieren, wodurch der Nenner des ersten Terms näherungsweise zu 1,0 mm³ - 0,5 mm³ = 0,5 mm³ wird. Dabei entsteht ein Höhenunterschied am Rand von 15 µm, was praktikabel ist, da dies beim SMILE-Verfahren ohnehin der Regelfall ist. Der zweite Term kann nicht gewinnbringend vergrößert werden. Zwar sind Vergrößerungen von *S_{S}* theoretisch möglich, müssten aber durch größere S_{A} ausgeglichen werden. Der dritte Term ist ebenfalls vorbestimmt. Man kann also für diese beispielhaften Überlegungen (mit der Annahme S_{S} = 0) auch schreiben ${S}_{A} \leq + 1 , 79 dpt + 1 , 46 {{C}_{S}}^{-}$

Für die stets negative Größe *S_{A}* erhält man somit als Bedingung, dass das Volumen des Implantats 26 nicht größer als das Volumen des zuvor entfernten bzw. entnommenen Lentikels 21 (mit gleichem Durchmesser der optischen Zone) ist. Fig. 6 zeigt dies aufgrund ihrer schematischen Darstellung nicht.

Wenn, wie hier beschrieben, das Implantat so gewählt wird, dass diese Bedingung eingehalten wird, kann es sein, dass (abweichend von der Beschreibung oben) aus dem Set nicht das Implantat 26 mit dem unmittelbar benachbarten Brechkraftwert gewählt wird. Man wählt dann das Implantat 26, mit welchem die obige Bedingung näherungsweise am besten erfüllt wird.

Als Beispiel soll ein hyperopes Auge mit S = +2,0 dpt und C = +1,5 dpt vollständig korrigiert werden (S_{T} = -2,0 dpt, C_{T} = -1,5 dpt). Es ist also B_{T_Max} = 0 - (+2,0 dpt + +1,5 dpt) = -3,5 dpt und B_{T_Min} = -20 dpt. Aus einem Satz von sphärischen Implantaten wird das Implantat 26 mit S₄ = +4,0 dpt ausgewählt (Schritt S2 in Fig.7).

Für die subtraktive Sphäre ergibt sich S_{S} = B_{T_Max} - Sᵢ = -3,5 dpt + +4,0 dpt = -0,5 dpt und für die Zylinderkomponente C_{S} = C _{T}= +1,5 dpt. Das subtraktiv entfernte bzw. entnommene Lentikel 21 würde für sich alleine also eine Myopie mit S = -0,5 dpt und C = -1,5 dpt perfekt korrigieren.

Seine Berechnung und Erzeugung im Auge des Patienten ist dem Fachmann bekannt. Seine chirurgische Entfernung bzw. Entnahme bewirkt temporär eine Verstärkung der bestehenden Hyperopie zu S = +4,0 dpt bei gleichzeitig vollständiger Korrektur des Zylinders (C = 0 dpt). Die verbleibende rein sphärische Hyperopie wird nun durch die Eingliederung des ausgewählten Implantats 26 korrigiert. Die Anwendung der obigen Ungleichung mit *C_{S}*⁻ = +1,5 *dpt* ergibt ${S}_{A} \leq + 3.98 dpt$

Diese Bedingung ist in durch Sᵢ = +4,0 dpt näherungsweise erfüllt. Dies genügt grundsätzlich, wobei Abweichungen von ± 20%, bevorzugt ± 10%, besonders bevorzugt ± 5% zulässig sein können. Die durch das Implantat 26 verursachte Dehnung des Caps 25 ist dann sehr gering. Auf Entlastungsschnitte kann verzichtet werden.

Man kann eine unterschiedlich große optische Zone akzeptieren, solange die kleinere der optischen Zonen groß genug ist, um die mesopische Pupille zu überdecken. Dadurch kann der Korrekturbereich ausgedehnt werden.

Alle diese Realisierungen beruhen auf der Grundidee, eine additive und eine subtraktive Korrektur zu kombinieren. Die Ausführungsvariante anhand des Volumenvergleichs ist eine beispielhafte und bevorzugte Ausführung. Überlegungen gleicher Art bezüglich winkelabhängiger Bogenlängen sind für den Fachmann analog durchführbar. Insbesondere zur genaueren Vorhersage des Restastigmatismus und entsprechende Gegenmaßnahmen zur Minimierung desselben ist ein solcher Ansatz ergiebiger als die Volumenberechnung.

Das Implantat kann aus Spendergewebe oder eine künstlichen Gewebematerial bestehen.

Der Anwender kann sich ein Element des Sets auch selbst aus einem geeigneten Rohling herstellen. Die Fertigung in Standardgrößen aus einem Rohling ist einfacher, als das Erzeugen eines patientenindividuellen Sonderimplantats. Auf diese Weise werden die Fertigung und Logistik radikal vereinfacht und das biomechanische Problem wird zumindest theoretisch exakt lösbar. Hierfür kann das vom Anwender hergestellte Inlay auch eine Zylinderkomponente oder andere Komponenten höherer Ordnung beinhalten. Damit erfordert die Implantation allerding eine achsrichtige Implantation. Methoden zur Unterstützung (Marken, Formpassungen, usw.) sind hierzu bekannt.

## Patentansprüche

1. Verfahren zum Erzeugen von Steuerdaten zur Korrektur der Refraktion eines Auges (2) durch Hornhautmodifikation, umfassend
- Empfangen von Daten über einen Refraktionskorrekturbedarf des Auges (2),
- Bestimmen, auf Basis des Refraktionskorrekturbedarfs, eines additiven Refraktionsveränderungswertes durch ein in die Hornhaut (17) einzusetzendes Implantat (26),
- Bestimmen eines subtraktiven Refraktionsveränderungswertes durch ein aus der Hornhaut (17) zu entfernendes Lentikel (21),
- wobei der additive Refraktionsveränderungswert zusammen mit dem subtraktiven Refraktionsveränderungswert den Refraktionskorrekturbedarf ergibt,
- Berechnen eines in der Hornhaut (17) zu isolierenden Lentikels (21) auf Basis des subtraktiven Refraktionsveränderungswertes derart, dass das Lentikel (21) ausgebildet ist, durch Entfernen aus der Hornhaut (17) den subtraktiven Refraktionsveränderungswert zu bewirken, und
- Berechnen einer Schnittfläche (22, 23) derart, dass sie in der Hornhaut (17) das Lentikel (21) isoliert und zugleich eine Kammer für das einzusetzende Implantat (26) umgrenzt, und Erzeugen von die Schnittfläche (22, 23) beschreibenden Daten,
- wobei das Lentikel (21) und das Implantat (26) jeweils in der Mitte dicker sind als am Rand oder das Lentikel (21) und das Implantat (26) jeweils in der Mitte dünner sind als am Rand.

2. Verfahren nach Anspruch 1, wobei der subtraktive Refraktionsveränderungswert aus dem Unterschied zwischen dem Refraktionskorrekturbedarf und dem zuvor bestimmten additiven Refraktionsveränderungswert bestimmt wird.

3. Verfahren nach einem der obigen Ansprüche, wobei der additive Refraktionsveränderungswert eine Überkorrektur einer Hyperopie oder Myopie bewirkt und der subtraktive Refraktionsveränderungswert eine dazu gegenläufige Myopie- bzw. Hyperopiekorrektur umfasst.

4. Verfahren nach einem der obigen Ansprüche, wobei der additive Refraktionsveränderungswert derart bestimmt wird, dass eine vorgegebene Maximalabweichung zwischen additivem Refraktionsveränderungswert und Refraktionskorrekturbedarf nicht überschritten wird.

5. Verfahren nach einem der obigen Ansprüche, wobei das einzusetzende Implantat (26) multifokal ist, um eine Presbyopie-Korrektur zu bewirken.

6. Verfahren nach einem der obigen Ansprüche, weiter umfassend Bereitstellen eines Sets von Implantaten (26), die jeweils zur additiven Refraktionsveränderung durch Einsetzen in die Hornhaut (17) ausgebildet sind, wobei das Set mehrere Implantate (26) mit individuellem additiven Refraktionsveränderungswert umfasst und wobei der Schritt des Bestimmens des Implantats (26) ein Auswählen eines der Implantate (26) umfasst, wobei optional im Auswahlschritt auf Basis des Refraktionskorrekturbedarfs aus dem Set dasjenige Implantat (26) ausgewählt wird, dessen additiven Refraktionsveränderungswert dem Refraktionskorrekturbedarf am nächsten liegt.

7. Verfahren nach einem der obigen Ansprüche, wobei das Volumen des Lentikels (21) größer oder gleich dem Volumen des Implantats (26) ist.

8. Vorrichtung zum Erzeugen von Steuerdaten zur Korrektur der Refraktion eines Auges (2) durch Hornhautmodifikation, umfassend
- eine Schnittstelle (29) zum Empfangen von Daten über einen Refraktionskorrekturbedarf des Auges (2),
- eine Berechnungseinrichtung (16a), die mit der Schnittstelle (29) verbunden und konfiguriert ist
-- zum Empfangen der Daten über den Refraktionskorrekturbedarf,
-- zum Bestimmen, auf Basis des Refraktionskorrekturbedarfs, eines additiven Refraktionsveränderungswertes durch ein in die Hornhaut (17) einzusetzendes Implantat (26),
-- zum Bestimmen eines subtraktiven Refraktionsveränderungswertes durch ein aus der Hornhaut (17) zu entfernendes Lentikel (21),
-- wobei der additive Refraktionsveränderungswert zusammen mit dem subtraktiven Refraktionsveränderungswert den Refraktionskorrekturbedarf ergibt,
-- zum Berechnen eines in der Hornhaut (17) zu isolierenden Lentikels (21) auf Basis des subtraktiven Refraktionsveränderungswertes derart, dass das Lentikel (21) ausgebildet ist, durch Entfernen aus der Hornhaut (17) den subtraktiven Refraktionsveränderungswert zu bewirken, und
-- zum Berechnen einer Schnittfläche (29) derart, dass sie in der Hornhaut (17) das Lentikel (21) isoliert und zugleich eine Kammer für das einzusetzende Implantat (26) umgrenzt, und zum Erzeugen von die Schnittfläche (29) beschreibenden Daten,
-- wobei das Lentikel (21) und das Implantat (26) jeweils in der Mitte dicker sind als am Rand oder das Lentikel (21) und das Implantat (26) jeweils in der Mitte dünner sind als am Rand.

9. Vorrichtung nach Anspruch 8, wobei die Berechnungseinrichtung (16a) konfiguriert ist, den subtraktiven Refraktionsveränderungswert aus dem Unterschied zwischen dem Refraktionskorrekturbedarf und dem zuvor bestimmten additiven Refraktionsveränderungswert zu bestimmen.

10. Vorrichtung nach einem der Ansprüche 8 oder 9, wobei
- die Berechnungseinrichtung (16a) konfiguriert ist, die beiden Refraktionsveränderungswerte derart zu bestimmen, dass der additive Refraktionsveränderungswertes eine Überkorrektur einer Hyperopie oder Myopie bewirkt und die subtraktiven Refraktionsveränderungswert eine dazu gegenläufige Myopie- bzw. Hyperopiekorrektur umfasst und/oder
- die Berechnungseinrichtung (16a) konfiguriert ist, den additiven Refraktionsveränderungswert derart zu bestimmen, dass eine vorgegebene Maximalabweichung zwischen additivem Refraktionsveränderungswert und Refraktionskorrekturbedarf nicht überschritten ist.

11. Vorrichtung nach einem der Ansprüche 8 bis 10, wobei das einzusetzende Implantat (26) multifokal ist, um eine Presbyopie-Korrektur zu bewirken.

12. Vorrichtung nach einem der Ansprüche 8 bis 11, wobei das Volumen des Lentikels (21) größer oder gleich dem Volumen des Implantats (26) ist.

13. System aus einer Vorrichtung nach einem der Ansprüche 8 bis 12 und einem Set von Implantaten (26), die jeweils zur additiven Refraktionsveränderung durch Einsetzen in die Hornhaut (17) ausgebildet sind, wobei das Set mehrere Implantate (26) mit individuellem additiven Refraktionsveränderungswert umfasst und wobei die Vorrichtung eine Schnittstelle (29) zum Empfangen von Daten über das Set von Implantaten (26) umfasst, wobei die Daten die individuellen additiven Refraktionsveränderungswerte umfassen und wobei die Berechnungseinrichtung konfiguriert ist, beim Bestimmen des additiven Refraktionsveränderungswertes eines der Implantate (26) aus dem Set auszuwählen.

14. System nach Anspruch 13, wobei die Implantate (26) des Sets jeweils eine rein rotationssymmetrische, insbesondere sphärische, Refraktionsveränderung bewirken und die Berechnungseinrichtung konfiguriert ist, das Lentikel (21) derart zu berechnen, dass es eine rotationsasymmetrische, insbesondere astigmatische, Refraktionsveränderung bewirkt, wobei optional die Berechnungseinrichtung konfiguriert ist, auf Basis des Refraktionskorrekturbedarfs aus dem Set dasjenige Implantat (26) auszuwählen, dessen Refraktionsveränderungswert dem Refraktionskorrekturbedarf am nächsten liegt.

15. Computerprogrammprodukt mit Programmcode, welcher, wenn in einen Computer geladen, ein Verfahren nach einem der Ansprüche 1 bis 7 zur Ausführung bringt.

## Claims

1. A method for generating control data for correcting the refraction of an eye (2) through corneal modification, comprising
- receiving data regarding a refractive correction requirement of the eye (2),
- determining, based on the refractive correction requirement, an additive refractive change value to be provided by an implant (26) to be inserted into the cornea (17),
- determining a subtractive refractive change value provided by a lenticule (21) to be removed from the cornea (17),
- wherein the additive refractive change value, together with the subtractive refractive change value, results in the refractive correction requirement,
- computing a lenticule (21) to be isolated within the cornea (17) based on the subtractive refractive change value, such that the lenticule (21) is configured to effect the subtractive refractive change value upon removal from the cornea (17), and
- computing a cut surface (22, 23) such that it isolates the lenticule (21) within the cornea (17) and simultaneously defines a chamber for the implant (26) to be inserted, and generating data defining the cut surface (22, 23),
- wherein the lenticule (21) and the implant (26) are each thicker in the center than at the edge, or the lenticule (21) and the implant (26) are each thinner in the center than at the edge.

2. The method according to claim 1, wherein the subtractive refractive change value is determined from the difference between the refractive correction requirement and the previously determined additive refractive change value.

3. The method according to any of the preceding claims, wherein the additive refractive change value causes overcorrection of hyperopia or myopia, and the subtractive refractive change value comprises a counteracting myopia or hyperopia correction, respectively.

4. The method according to any of the preceding claims, wherein the additive refractive change value is determined such that a predetermined maximum deviation between the additive refractive change value and the refractive correction requirement is not exceeded.

5. A method according to any of the preceding claims, wherein the implant (26) to be inserted is multifocal to provide correction for presbyopia.

6. The method according to any of the preceding claims, further comprising providing a set of implants (26), each of which is configured to provide an additive refractive change when inserted into the cornea (17), wherein the set comprises multiple implants (26) having individualized additive refractive change values, and wherein the step of determining the implant (26) comprises selecting one of the implants (26), wherein, optionally, in the selection step, based on the refractive correction requirement, the implant (26) from the set whose additive refractive change value is closest to the refractive correction requirement is selected.

7. The method according to any of the preceding claims, wherein the volume of the lenticule (21) is greater than or equal to the volume of the implant (26).

8. An apparatus for generating control data for correcting the refraction of an eye (2) through corneal modification, comprising
- an interface (29) for receiving data regarding a refractive correction requirement of the eye (2),
- a computing device (16a) connected to the interface (29) and configured
-- to receive the data regarding the refractive correction requirement,
-- to determine, based on the refractive correction requirement, an additive refractive change value provided by an implant (26) to be inserted into the cornea (17),
-- to determine a subtractive refractive change value provided by a lenticule (21) to be removed from the cornea (17),
-- wherein the additive refractive change value, together with the subtractive refractive change value, yields the refractive correction requirement,
-- to compute a lenticule (21) to be isolated within the cornea (17) based on the subtractive refractive change value, such that the lenticule (21) is configured to effect the subtractive refractive change value upon removal from the cornea (17), and
-- to compute a cutting surface (29) such that it isolates the lenticule (21) within the cornea (17) and simultaneously defines a chamber for the implant (26) to be inserted, and to generate data describing the cutting surface (29),
-- wherein the lenticule (21) and the implant (26) are each thicker in the center than at the edge, or the lenticule (21) and the implant (26) are each thinner in the center than at the edge.

9. The apparatus according to claim 8, wherein the computing device (16a) is configured to determine the subtractive refractive change value from the difference between the refractive correction requirement and the previously determined additive refractive change value.

10. The apparatus according to any one of claims 8 or 9, wherein
- the computing device (16a) is configured to determine the two refractive change values such that the additive refractive change value causes overcorrection of hyperopia or myopia and the subtractive refractive change value comprises a correction for myopia or hyperopia in the opposite direction, and/or
- the computing device (16a) is configured to determine the additive refractive change value such that a predetermined maximum deviation between the additive refractive change value and the refractive correction requirement is not exceeded.

11. The apparatus according to any one of claims 8 through 10, wherein the implant (26) to be inserted is multifocal to provide presbyopia correction.

12. The apparatus according to any one of claims 8 through 11, wherein the volume of the lenticule (21) is greater than or equal to the volume of the implant (26).

13. A system comprising an apparatus according to any one of claims 8 through 12 and a set of implants (26), each of which is configured to provide additive refractive change when inserted into the cornea (17), wherein the set comprises a plurality of implants (26) with individualized additive refractive change values, and wherein the apparatus comprises an interface (29) for receiving data regarding the set of implants (26), wherein the data comprises the individualized additive refractive power values, and wherein the computing means is configured to select one of the implants (26) from the set when determining the additive refractive power value.

14. A system according to claim 13, wherein the implants (26) of the set each produce a purely rotationally symmetric, in particular spherical, refractive change, and the computing device is configured to calculate the lenticule (21) such that it produces a rotationally asymmetric, in particular astigmatic, refractive change, wherein, optionally, the computing device is configured to select based on the refractive correction requirement, from the set, the implant (26) whose refractive change value most closely matches the refractive correction requirement .

15. A computer program product comprising program code which, when loaded into a computer, causes a method according to any one of claims 1 through 7 to be performed.

## Revendications

1. Procédé de génération de données de commande destinées à corriger la réfraction d'un œil (2) par modification de la cornée, comprenant
- la réception de données relatives à un besoin de correction de la réfraction de l'œil (2),
- la détermination, sur la base du besoin de correction de la réfraction, d'une valeur additive de modification de la réfraction par un implant (26) à insérer dans la cornée (17),
- la détermination d'une valeur de modification de réfraction soustractive par un lenticule (21) à retirer de la cornée (17),
- la valeur de modification de réfraction additive, combinée à la valeur de modification de réfraction soustractive, donnant le besoin de correction de réfraction,
- calcul d'un lenticule (21) à isoler dans la cornée (17) sur la base de la valeur de modification réfractive soustractive, de telle sorte que le lenticule (21) soit conçu pour produire, par son retrait de la cornée (17), la valeur de modification réfractive soustractive, et
- calculer une surface de coupe (22, 23) de telle sorte qu'elle isole le lenticule (21) dans la cornée (17) tout en délimitant une chambre destinée à l'implant (26) à insérer, et générer des données décrivant la surface de coupe (22, 23),
- le lenticule (21) et l'implant (26) étant chacun plus épais au centre qu'à la périphérie, ou le lenticule (21) et l'implant (26) étant chacun plus minces au centre qu'à la périphérie.

2. Procédé selon la revendication 1, dans lequel la valeur de modification réfractive soustractive est déterminée à partir de la différence entre le besoin de correction réfractive et la valeur de modification réfractive additive préalablement déterminée.

3. Procédé selon l'une des revendications précédentes, dans lequel la valeur de modification de réfraction additive entraîne une surcorrection d'une hypermétropie ou d'une myopie, et la valeur de modification de réfraction soustractive comprend une correction de myopie ou d'hypermétropie de sens opposé.

4. Procédé selon l'une des revendications ci-dessus, dans lequel la valeur de modification de la réfraction additive est déterminée de telle sorte qu'un écart maximal prédéfini entre la valeur de modification de la réfraction additive et le besoin de correction réfractive ne soit pas dépassé.

5. Procédé selon l'une des revendications ci-dessus, dans lequel l'implant (26) à mettre en place est multifocal afin d'assurer une correction de la presbytie.

6. Procédé selon l'une des revendications ci-dessus, comprenant en outre la mise à disposition d'un ensemble d'implants (26) qui sont chacun conçus pour provoquer une modification réfractive additive par insertion dans la cornée (17), l'ensemble comprenant plusieurs implants (26) présentant chacun une valeur de modification réfractive additive individuelle, et l'étape de détermination de l'implant (26) comprend la sélection de l'un des implants (26), l'implant (26) dont la valeur de modification réfractive additive est la plus proche du besoin de correction réfractive étant sélectionné, de manière facultative, à partir de l'ensemble lors de l'étape de sélection, en fonction dudit besoin de correction réfractive.

7. Procédé selon l'une des revendications ci-dessus, dans lequel le volume du lenticule (21) est supérieur ou égal au volume de l'implant (26).

8. Dispositif destiné à générer des données de commande pour la correction de la réfraction d'un œil (2) par modification cornéenne, comprenant
- une interface (29) destinée à recevoir des données relatives à un besoin de correction réfractive de l'œil (2),
- un dispositif de calcul (16a) relié à l'interface (29) et configuré
-- pour recevoir les données relatives au besoin de correction de la réfraction,
-- pour déterminer, sur la base du besoin de correction de la réfraction, une valeur de modification de la réfraction additive par un implant (26) à insérer dans la cornée (17),
-- pour déterminer une valeur de modification de réfraction soustractive par un lenticule (21) à retirer de la cornée (17),
-- la valeur de modification de réfraction additive, combinée à la valeur de modification de réfraction soustractive, donnant le besoin de correction de réfraction,
-- pour calculer un lenticule (21) à isoler dans la cornée (17) sur la base de la valeur de modification de réfraction soustractive, de telle sorte que le lenticule (21) soit conçu pour produire, par son retrait de la cornée (17), la valeur de modification de réfraction soustractive, et
-- pour calculer une surface de coupe (29) de telle sorte qu'elle isole le lenticule (21) dans la cornée (17) tout en délimitant une chambre destinée à l'implant (26) à insérer, et pour générer des données décrivant la surface de coupe (29),
-- le lenticule (21) et l'implant (26) étant chacun plus épais au centre qu'à la périphérie, ou le lenticule (21) et l'implant (26) étant chacun plus minces au centre qu'à la périphérie.

9. Dispositif selon la revendication 8, dans lequel le moyen de calcul (16a) est configuré pour déterminer la valeur de modification de réfraction soustractive à partir de la différence entre le besoin de correction de réfraction et la valeur de modification de réfraction additive préalablement déterminée.

10. Dispositif selon l'une des revendications 8 ou 9, dans lequel
- le dispositif de calcul (16a) est configuré pour déterminer les deux valeurs de modification de la réfraction de telle sorte que la valeur de modification de la réfraction additive entraîne une surcorrection d'une hypermétropie ou d'une myopie et que la valeur de modification de la réfraction soustractive comprenne une correction de myopie ou d'hypermétropie opposée à celle-ci et/ou
- le dispositif de calcul (16a) est configuré pour déterminer la valeur de variation de réfraction additive de telle sorte qu'un écart maximal prédéfini entre la valeur de variation de réfraction additive et le besoin de correction de réfraction ne soit pas dépassé.

11. Dispositif selon l'une des revendications 8 à 10, dans lequel l'implant (26) à mettre en place est multifocal afin d'assurer une correction de la presbytie.

12. Dispositif selon l'une des revendications 8 à 11, dans lequel le volume du lenticule (21) est supérieur ou égal au volume de l'implant (26).

13. Système composé d'un dispositif selon l'une des revendications 8 à 12 et d'un ensemble d'implants (26) conçus chacun pour apporter une modification réfractive additive par insertion dans la cornée (17), l'ensemble comprenant plusieurs implants (26) présentant chacun une valeur de modification réfractive additive individuelle, et le dispositif comprenant une interface (29) destinée à recevoir des données concernant le jeu d'implants (26), lesdites données comprenant les valeurs individuelles de modification réfractive additive, et le dispositif de calcul étant configuré pour sélectionner, lors de la détermination de la valeur de modification réfractive additive, l'un des implants (26) du jeu.

14. Système selon la revendication 13, dans lequel les implants (26) de l'ensemble provoquent chacun une modification de la réfraction purement symétrique en rotation, en particulier sphérique, et le dispositif de calcul est configuré pour calculer le lenticule (21) de telle sorte qu'il provoque une modification de la réfraction asymétrique en rotation, en particulier astigmatique, , le dispositif de calcul étant en outre configuré, de manière facultative, pour sélectionner, à partir du kit et en fonction du besoin de correction réfractive, l'implant (26) dont la valeur de modification de la réfraction est la plus proche de ce besoin.

15. Produit logiciel comprenant un code de programme qui, lorsqu'il est chargé dans un ordinateur, met en œuvre un procédé selon l'une des revendications 1 à 7.
